# EUROPÄISCHE PATENTANMELDUNG

(11) **EP 4 137 804 A1**
(43) Veröffentlichungstag der Anmeldung: **22.02.2023**
(21) Anmeldenummer: 22190772.8
(22) Anmeldetag: 17.08.2022
(51) Int. Cl.: G01N 25/72, G01N 21/00, G01N 29/00, G01N 33/02

(54) **VERFAHREN UND SYSTEM ZUR MANUELLEN QUALITÄTSPRÜFUNG VON OBST UND GEMÜSE UND ANDEREN LEBENSMITTELN**

(30) Priorität: 20.08.2021 DE 102021209197; 13.10.2021 DE 102021211546
(71) Anmelder: Fraunhofer-Gesellschaft zur Förderung der angewandten Forschung e.V., 80686 München (DE); Hochschule für Technik und Wirtschaft des Saarlandes, 66117 Saarbrücken (DE)
(72) Erfinder: Osman, Ahmad, 66123 Saarbrücken (DE); Albert-Weiß, Dominique, 66123 Saarbrücken (DE)
(74) Vertreter: Pfenning, Meinig & Partner mbB

(57) **Zusammenfassung**

Die Erfindung betrifft ein Verfahren und ein System (10) zur manuellen Qualitätsprüfung von Obst und Gemüse und anderen Lebensmitteln, wobei das System (10) aufweist:
- einen Kamerasensor (28) zum Erfassen von Bilddaten (B) des Lebensmittels auf Basis elektromagnetischer Strahlung im sichtbaren Wellenlängenbereich;
- einen Thermografiesensor (26) zum Erfassen von Thermografiedaten (T) des Lebensmittels;
- eine Einrichtung (30) zum Erfassen von Schwingungsdaten (S), die ein Schwingungsverhalten des Lebensmittels beschreiben;
- wenigstens eine Ermittlungseinrichtung (21), die dazu eingerichtet ist, auf Basis der Bilddaten (B), Thermografiedaten (T) und Schwingungsdaten (S) sowie mittels Maschinenlernmodellsystems (100) mit wenigstens einem Maschinenlernmodell (54-60, 62, 63) eine Qualitätsgröße für das Lebensmittel zu ermitteln;

wobei das System (10) einen handhaltbaren Teil (14) und einen stationären Teil (12) umfasst, wobei der handhaltbare Teil (14) aktiv relativ zu dem Lebensmittel und zu dem stationären Teil (12) beweglich ist,
und wobei der handhaltbare Teil (14) zumindest einen von dem Kamerasensor (28), dem Thermografiesensor (26) und der Einrichtung (30) zum Erfassen der Schwingungsdaten (S) aufweist.

## Beschreibung

Die Erfindung betrifft ein Verfahren und ein System zur manuellen Qualitätsprüfung von Obst und Gemüse und anderen Lebensmitteln, insbesondere von landwirtschaftlichen Erzeugnissen, wie zum Beispiel Fisch oder Fleisch.

Ein zentrales Thema in der Lebensmittelüberwachung und -kontrolle ist die Bewertung der Qualität von landwirtschaftlichen Erzeugnissen wie Obst und Gemüse mit Hilfe zerstörungsfreier Prüfverfahren. Innerhalb der landwirtschaftlichen Lieferkette werden bisher meist visuelle Inspektionen durch Personen durchgeführt, die jedoch entsprechend subjektiv sind.

Alternativ existieren technische Systeme, die mittels in der Regel einer einzigen Sensorart eine definierte Eigenschaft des Lebensmittels erfassen und darauf basierend zum Beispiel per Schwellenwertabgleich eine Qualitätsaussage treffen.

Derartige technische Systeme existieren in handhaltbarer Form, sodass ein Benutzer manuell die Prüfung des Lebensmittels steuern und durchführen kann. Alternativ sind fest installierte Systeme zur automatischen Lebensmittelprüfung bekannt. Beispielsweise können derartige Systeme entlang von Transportpfaden der Lebensmittel installiert sein und diese automatisch erfassen. Beispielsweise können sie entlang von Förderbändern in Sortier- oder Verpackungsanlagen fest installiert sein.

Als ein Messprinzip der Lebensmittelprüfung ist eine akustische Prüfung bekannt, bei der Schwingungseigenschaften des Lebensmittels erfasst werden, insbesondere auf Basis oder in Form von akustischen Signalen. Diese Signale können beispielsweise durch Vibrationen des Lebensmittels infolge einer Schwingungsanregung hervorgerufen werden. Letztere umfasst meist einen impulsartigen Stoß oder eine Anregung durch Schallwellen.

Insbesondere können akustische Resonanzfrequenzen des Lebensmittels sensorisch erfasst werden. So offenbart die US 2007/0079644 A1 einen Reifedetektor für Melonen, der erwartete akustische Resonanzfrequenzen mit gemessenen Resonanzfrequenzen vergleicht und darauf basierend als Qualitätsmerkmal einen Reifegrad bestimmt.

Die US 6,276,536 B1 lehrt eine Bestimmung des Reifegrads einer Frucht anhand von auf Basis einer Schwingungsanregung ermittelten Dämpfungsverhältnissen und Elastizitätswerten sowie mittels einer Schwellenwertbetrachtung.

Weiter existieren Lösungen, die ein Lebensmittel mittels für den sichtbaren Wellenlängenbereich sensitiven Kamerasensoren (siehe zum Beispiel CN 102654463 A) oder mittels Thermografiesensoren erfassen (siehe zum Beispiel Gurupatham, S. K., Ilksoy, E., Jacob, N., Van der Horn, K., Fahad, F.: Fruit Ripeness Estimation for Avocado Using Thermal Imaging, ASME 2018 International Mechanical Engineering Congress and Exposition, 2018).

Die vorstehenden Lösungen besitzen jedoch nur ein begrenztes Anwendungsspektrum, da sie nur für eine bestimmte Obst- oder Gemüsesorte verwendbar sind. Zudem kann die Aussagekraft des Prüfungsergebnisses dadurch beeinträchtigt sein, dass der Zusammenhang zwischen den ermittelten Sensordaten und dem Reifegrad nicht eindeutig ist, beispielsweise da er von anderen nicht berücksichtigten Größen abhängt.

Weiter existieren Prüfungsmethoden, die sich auf eine Visualisierung eines sensorischen Signals bei der Überprüfung des Lebensmittels beschränken und die Beurteilung der Fruchtqualität der subjektiven Interpretation durch den Benutzer überlassen. Dies ist insbesondere dann nachteilig, wenn die sensorischen Signale keinen direkten Bezug zu einer eigentlich gesuchten Qualitätsgröße (zum Beispiel dem Reifegrad) aufweisen und dieser Zusammenhang subjektiv beurteilt werden muss.

Gerade für den Anwendungsfall mobiler (d. h. nicht in einer technischen Anlage fest installierter) und/oder manuell zu betätigender Systeme hat sich gezeigt, dass sich mit den bisherigen Lösungen nicht immer ausreichend zuverlässige Qualitätsaussagen erzielen lassen. Wie erwähnt, ist auch das Anwendungsspektrum existierender Systeme meist nur auf eine Lebensmittelart und insbesondere eine einzige Gemüse- oder Obstsorte beschränkt.

Die Erfindung richtet sich daher auf die Aufgabe, eine Lösung zur sensorisch gestützten manuellen Qualitätsüberprüfung von Lebensmitteln bereitzustellen, die sich durch eine verbesserte Genauigkeit und ein vergrößertes Anwendungsspektrum auszeichnet.

Diese Aufgabe wird durch die Gegenstände der beigefügten unabhängigen Ansprüche gelöst. Vorteilhafte Weiterbildungen sind in den abhängigen Ansprüchen angegeben.

Es wird ein System zur manuellen Qualitätsprüfung von Obst und Gemüse und anderen Lebensmitteln vorgeschlagen, mit:
- einem Kamerasensor zum Erfassen von Bilddaten des Lebensmittels auf Basis elektromagnetischer Strahlung im sichtbaren Wellenlängenbereich;
- einen Thermografiesensor zum Erfassen von Thermografiedaten des Lebensmittels;
- einer Einrichtung zum Erfassen von Schwingungsdaten, die ein Schwingungsverhalten des Lebensmittels beschreiben;
- wenigstens einer Ermittlungseinrichtung, die dazu eingerichtet ist, auf Basis der Bilddaten, Thermografiedaten und Schwingungsdaten sowie mittels eines Maschinenlernmodellsystems mit wenigstens einem Maschinenlernmodell eine Qualitätsgröße für das Lebensmittel zu ermitteln.

Bevorzugt umfasst das System einen handhaltbaren Teil und einen stationären Teil, wobei der handhaltbare Teil aktiv relativ zu dem Lebensmittel und zu dem stationären Teil beweglich ist.

Der handhaltbare Teil weist vorzugsweise wenigstens einen von der Einrichtung zum Erfassen der Schwingungsdaten des Lebensmittels, dem Kamerasensor oder dem Thermografiesensor auf. Ferner bevorzugt weist der stationäre Teil wenigstens einen entsprechend anderen von der Einrichtung zum Erfassen der Schwingungsdaten des Lebensmittels, dem Kamerasensor oder dem Thermografiesensor auf.

Ebenso offenbart wird ein System der vorstehenden Art, das lediglich zwei von Kamerasensor, Thermografiesensor und Ermittlungseinrichtung aufweist. Dieses System kann gemäß jeglicher hierin geschilderter Variante ausgestaltet sein und weitergebildet werden.

Das hierin offenbarte System zeichnet sich allgemein dadurch aus, dass es eine Mehrzahl unterschiedlicher Sensorsysteme und insbesondere unterschiedlicher Sensorprinzipien (d. h. Messprinzipien) vereint, deren jeweilige Sensordaten (oder auch Messdaten) bei dem Bestimmen der Qualitätsgröße berücksichtigt werden. Anders ausgedrückt erfolgt eine multimodale Qualitätsgrößenermittlung basierend auf einer Mehrzahl verschiedenartiger Sensoren und verschiedenartiger Sensordaten. Im Rahmen dieser Offenbarung kann die vorstehend genannte Einrichtung zum Erfassen von Schwingungsdaten ebenfalls als ein Sensor und können die Schwingungsdaten ebenfalls als Sensordaten verstanden werden.

Es wurde erkannt, dass die verschiedenartigen Sensoren und Sensordaten einen aussagekräftigen Rückschluss auf die Qualität des Lebensmittels ermöglichen oder, anders ausgedrückt, komplementär zueinander sind. Beispielsweise lassen sich hierdurch Abhängigkeiten einer gesuchten Qualitätsgröße (insbesondere eines Reifegrads) von mehreren unterschiedlichen Lebensmitteleigenschaften berücksichtigen. Dies beruht auf der Erkenntnis, dass die Lebensmittelqualität in der Regel nicht anhand nur einer einzigen Messgröße aussagekräftig bewertet werden kann, sondern meist von mehreren unterschiedlichen Messgrößen abhängt und insbesondere einen nichtlinearen Zusammenhang zu diesen Messgrößen aufweist.

Dem wird vorliegend auch dadurch Rechnung getragen, dass ein Maschinenlernmodellsystem zum Einsatz kommt, welches entsprechende mehrfache Abhängigkeiten und/oder Nichtlinearitäten berücksichtigen und genauer gesagt abbilden und modellieren kann.

Bevorzugt gibt das System die Qualitätsgröße in quantifizierter und/oder klassifizierter Form aus (beispielsweise mittels einer Anzeigeeinrichtung). Folglich ist keine subjektive Interpretation von abstrakten Messergebnissen seitens des Benutzers erforderlich.

Wie hierin noch näher ausgeführt, ist das System vorzugsweise in der Lage, unterschiedliche Lebensmittelarten (beispielsweise der Obst- oder Gemüsesorten) zu erkennen und/oder zu berücksichtigen, was eine sortenspezifische Auswertung der Sensordaten und/oder das Ermitteln sortenspezifischer Qualitätsgrößen ermöglicht. Dies erweitert das Anwendungsspektrum.

Allgemein zeichnet sich das System durch einen kompakten Aufbau und ein leichtes Gewicht aus, was einen einfachen Transport und eine einfache Handhabung ermöglicht. Dies ermöglicht den mobilen Einsatz des Systems zum Beispiel im Freien und allgemein außerhalb von Produktionsräumen, Laboren oder Verpackungsanlagen.

Der Thermografiesensor kann eine Wärmebildkamera sein. Allgemein kann der Thermografiesensor dazu eingerichtet sein, als Thermografiedaten eine Strahlungsintensität von Infrarotstrahlung zu erfassen, die beispielsweise von dem zu überprüfenden Lebensmittel ausgeht. Die Thermografiedaten können in Form wenigstens eines Wärmebildes gespeichert werden.

Bevorzugt ist der Thermografiesensor ein aktiver Thermografiesensor, der eine Wärmequelle zum Beispiel in Form einer wärmestrahlenden Lampe umfasst (zum Beispiel einer Blitzlampe). In an sich bekannter Weise kann hierüber ein Wärmefluss und/oder eine Wärmeleitung der mittels der Wärmequelle in das zu untersuchende Lebensmittel eingebrachten Wärme erfasst werden, indem wiederum Thermografiedaten der oben genannten Art ermittelt werden. Beispielsweise können anhand von Inhomogenitäten des Wärmeflusses Defekte, insbesondere innere Defekte, des Lebensmittels erkannt werden.

Die Schwingungsdaten können Schwingungsfrequenzen und insbesondere Schwingungsfrequenzverläufe umfassen oder darauf basierend ermittelt werden. Insbesondere können als oder auf Basis der Schwingungsdaten Resonanzfrequenzen des Lebensmittels ermittelt werden, wenn dieses eine Schwingungsanregung erfährt. Die Schwingungsdaten können akustische Signale sein oder auf Basis akustischer Signale ermittelt werden. Die akustischen Signale können jeweils von dem schwingenden und insbesondere vibrierenden Lebensmittel ausgehen. Beispielsweise können die Schwingungsdaten auf Basis von Vibrationsschall des Lebensmittels ermittelt werden.

Bei der Ermittlungseinrichtung kann es sich um eine computerbasierte Steuereinrichtung handeln, die beispielsweise wenigstens einen Prozessor umfasst. Der Prozessor ist bevorzugt dazu eingerichtet, die erhaltenen Sensordaten (also die Thermografiedaten, Schwingungsdaten, Bilddaten und jegliche anderen hierin erläuterten und an die Ermittlungseinrichtung übermittelten sensorischen Daten) gemäß jeglichem der hierin geschilderten Ansätze zu verarbeiten, insbesondere unter Verwendung des Maschinenlernmodellsystems. Beispielsweise kann die Ermittlungseinrichtung dazu eingerichtet sein, ein Computerprogramm und/oder Computeralgorithmen auszuführen, die das Maschinenlernmodellsystem implementieren.

Das Maschinenlernmodellsystem kann wenigstens ein künstliches neuronales Netz als Beispiel eines einzelnen Maschinenlernmodells umfassen. Typischerweise weist das Maschinenlernsystem aber mehrere künstliche neuronale Netze auf. Allgemein kann es sich um einen Algorithmus und/oder zumindest einen Teil eines Computerprogramms handeln, der Verarbeitungsvorschriften definiert, um die Qualitätsgröße zu ermitteln. Insbesondere kann das Maschinenlernmodellsystem Zusammenhänge zwischen den Sensordaten und/oder darauf basierend erzeugter Eingangsdaten des Maschinenlernmodellsystems und der Qualitätsgröße definieren. Diese Zusammenhänge können im Rahmen eines maschinellen Lernvorgangs erlernt werden. Dieser Lernvorgang (auch als Training bezeichnet) kann ein sogenannter supervised-Lernvorgang unter Verwendung von Trainingsdatensätzen sein, die verifizierte Zusammenhänge aus Sensordaten und dabei vorliegenden Qualitätsgrößen enthalten.

Das System kann ferner einen Umgebungssensor zum Erfassen wenigstens einer Umgebungsgröße aufweisen, vorzugsweise der Temperatur und bzw. oder der Luftfeuchtigkeit. Besonders vorzugsweise ist der Umgebungssensor dazu eingerichtet, sowohl die Temperatur als auch die Luftfeuchtigkeit zu erfassen. Der Umgebungssensor kann alternativ als atmosphärischer Sensor und die Umgebungsgröße als kann alternativ als atmosphärische Größe bezeichnet werden. Der Umgebungssensor kann ein Thermo-Hygrometer sein. Die wenigstens eine Qualitätsgröße ist bevorzugt auch auf Basis von Erfassungsdaten des Umgebungssensors ermittelbar.

Zusätzlich oder alternativ kann das System einen Gewichtssensor zum Erfassen des Gewichts des Lebensmittels aufweisen. Die Qualitätsgröße ist bevorzugt auch auf Basis von Erfassungsdaten des Gewichtssensors ermittelbar.

Der Umgebungssensor und/oder der Gewichtssensor können von dem stationären Teil umfasst sein. Dies erhöht die Messgenauigkeit, da zum Beispiel die Gefahr von Wärmeübertragungen an den Umgebungssensor durch den Benutzer reduziert wird. Auch mindert dies die Komplexität und das Gewicht des beweglichen Teils.

Durch einen zusätzlichen Umgebungssensor und/oder Gewichtssensor kann die Ermittlungsgenauigkeit gesteigert werden. Beispielsweise können anhand des Umgebungssensors die Thermografiedaten verifiziert und/oder kalibriert werden. Der Gewichtssensor kann zusätzliche Hinweise auf den Reifegrad liefern, insbesondere wenn gleichzeitig auch morphologische Größen anhand der Bilddaten bestimmt werden. Beispielsweise können Abmessungen des Lebensmittels in Zusammenhang mit dessen Gewicht Rückschlüsse auf einen Reifegrad ermöglichen.

Gemäß einer bevorzugten Ausführungsform umfasst die Einrichtung zum Erfassen der Schwingungsdaten einen Aktor zum Ausüben eines Stoßes auf das Lebensmittel. Dieser Aktor kann zum Bespiel einen Stößel umfassen, der vorzugsweise selektiv bewegbar ist.

Zusätzlich oder alternativ kann die Einrichtung zum Erfassen der Schwingungsdaten wenigstens einen Mikrofonsensor zum Erfassen akustischer Signale des Lebensmittels infolge des Stoßes umfassen (d.h. zum Erfassen akustischer Signale, die von dem Lebensmittel z.B. infolge von dessen Vibrationen ausgehen). Diese Signale können die Schwingungsdaten bilden und/oder die Schwingungsdaten können auf Grundlage dieser Signale ermittelt werden. Es kann auch vorgesehen sein, ein Mikrofonarray zu verwenden, um gewisse Störsignale oder Hintergrundgeräusche besser herausfiltern zu können.

Gemäß einer Variante ist zumindest für eine Art der Sensordaten wenigstens ein gesondertes Maschinenlernmodell bereitgestellt, zum Beispiel ein neuronales Netz. Dies kann die Qualitätsgröße (zum Beispiel zumindest vorläufig) anhand dieser Sensordaten-Art ermitteln, zum Beispiel durch Lösen der nachstehende Klassifikations- und/oder Regressionsaufgabe. Wenn eine Mehrzahl entsprechend sensordatenspezifischer Maschinenlernmodelle bereitgestellt ist, können die jeweiligen Ermittlungsergebnisse anschließend zum Ermitteln eines Gesamtergebnisses (also beispielsweise zum Ermitteln einer finalen oder resultierenden Qualitätsgröße) fusioniert werden.

Zusammengefasst sieht eine Weiterbildung vor, dass das Maschinenlernmodellsystem eine Mehrzahl von Maschinenlernmodellen umfasst, von denen wenigstens eines dazu eingerichtet ist, auf Basis nur einer ausgewählten Art der Sensordaten ein Ergebnis (zum Beispiel eine vorläufige Qualitätsgröße) zu ermitteln, das mit einem ermittelten Ergebnis wenigstens eines anderen Maschinenlernmodells des Maschinenlernmodellsystems zum Bestimmen der (finalen) Qualitätsgröße fusionierbar ist.

Innerhalb des Maschinenlernmodellsystems und/oder eines Computerprogramms, das die Ermittlungseinrichtung zum Ermitteln der Qualitätsgröße ausführt, kann ein erstes Modul für eine Grobprüfung des Lebensmittels vorgesehen sein und ein zweites Modul für eine Feinprüfung und vorzugsweise Ermittlung der Qualitätsgröße. Die Module können Bestandteil des Maschinenlernmodellsystems sein. Jedes der Module, zumindest aber das zweite, kann wenigstens ein Maschinenlernmodell umfassen. Wenigstens eine Ausgangsgröße des ersten Moduls kann eine Eingangsgröße des zweiten Moduls bilden. Wenigstens eine Ausgangsgröße des ersten Moduls kann einem Benutzer angezeigt werden, beispielweise infolge eines manuellen Anforderns und/oder gleichzeitig zu einem Anzeigen der ermittelten Qualitätsgröße.

Die Qualitätsgröße kann einen Reifegrad des Lebensmittels angeben.

Die Qualitätsgröße kann eine voraussichtliche Haltbarkeit und/oder einen voraussichtlichen Zuckergehalt des Lebensmittels angeben.

Gemäß einer bevorzugten Variante wird unter Verwendung des Maschinenlernmodellsystems wenigstens ein Klassifizierungsvorgang durchgeführt, vorzugsweise um als Qualitätsgröße den Reifegrad des Lebensmittels zu klassifizieren.

Zusätzlich oder alternativ kann unter Verwendung des Maschinenlernmodellsystems wenigstens ein Regressionsvorgang durchgeführt werden, vorzugsweise um als Qualitätsgröße zum Beispiel die Haltbarkeit, den Zuckergehalt oder eine Rinden- oder Schalenstärke des Lebensmittels zu ermitteln.

Das Maschinenlernmodellsystem und insbesondere dessen einzelne Maschinenlernmodelle kann/können für das Lösen einer derartigen Klassifikations- und/oder Regressionsaufgabe trainiert und/oder eingerichtet sein.

Eine Weiterbildung sieht vor, dass die Ermittlungseinrichtung dazu eingerichtet ist, auf Basis der Bilddaten wenigstens einen der folgenden zu ermitteln:
einen Ort des Lebensmittels, an dem zum Erfassen des Schwingungsverhaltens eine Schwingungsanregung erfolgt;
einen Ort des Lebensmittels, an dem dieses eine vorbestimmte unnatürliche Oberflächenabweichung aufweist, beispielsweise einen Aufkleber oder eine Markierung;
einen Ort des Lebensmittels, an dem dieses einen vorbestimmten natürlichen Oberflächendefekt aufweist, beispielsweise einen Kratzer, Schimmel oder eine Druckstelle.

Jegliche der vorstehenden Orte können von einem ersten (Maschinenlernmodellsystem-) Modul der vorstehend geschilderten Art ermittelt und optional als eine Ausgangsgröße an ein geschildertes zweites Modul ausgegeben werden.

Allgemein kann die Qualitätsgröße unter Berücksichtigung der genannten Orte bzw. Positionen ermittelt werden, beispielsweise in dem diese Orte als Eingangsgrößen des Maschinenlernmodellsystems verwendet werden und/oder derartige Eingangsgrößen unter Berücksichtigung der Orte ermittelbar sind. Es hat sich gezeigt, dass sich hierdurch die Ermittlungsgenauigkeit der Qualitätsgröße erhöhen lässt, beispielsweise da dies eine genauere Unterscheidung zwischen natürlichen und unnatürlichen Oberflächenabweichungen bzw. - defekten ermöglicht und/oder eine Verifikation von Ermittlungsergebnissen des Maschinenlernmodellsystems ermöglicht.

Gemäß einer bevorzugten Ausführungsform ist das Maschinenlernmodellsystem eines bereits im Einsatz befindlichen oder ausgelieferten Systems anpassbar, insbesondere trainierbar. Insbesondere kann das System für ein aktives Lernen eingerichtet sein und/oder eine aktive Lernumgebung bereitstellen. Dies kann umfassen, dass auf Basis von Nutzereingaben, insbesondere in Form von Rückmeldungen auf Eingabeaufforderungen, unter Verwenden des Maschinenlernmodellsystems ermittelte Ergebnisse verifiziert werden und/oder Eigenschaften eines zu prüfenden Lebensmittels direkt abgefragt werden.

Die Eingabeaufforderungen können insbesondere bei einer unzulässig hohen Ermittlungsunsicherheit der Qualitätsgröße oder von Zwischenergebnissen des Maschinenlernmodellsystems ausgegeben werden, insbesondere wenn das Lebensmittel von dem Maschinenlernmodellsystem nicht automatisch klassifiziert werden kann.

Allgemein formuliert können im Rahmen des aktiven Lernens durch einen Benutzer bzw. dessen Eingaben Datensätze des Lebensmittels sowie mit dazugehörigen und von dem Maschinenlernmodellsystem prinzipiell zu ermittelnden Eigenschaften eingeben werden, d.h. kann der Benutzer für ein Erstellen und/oder Kennzeichnen (engl.: "labeln") derartiger Datensätze aufgefordert werden. Das Maschinenlernmodellsystem kann bevorzugt nach Erhalten einer definierten Mindestanzahl entsprechend aktiv gelernter Datensätze auf Basis dieser Datensätze neu trainiert werden.

Ebenso offenbart wird ein Verfahren zur Qualitätsprüfung von Obst und Gemüse und anderen Lebensmitteln, insbesondere ausgeführt mit einem System der hierin offenbarten Art, mit:
Erfassen von Bilddaten des Lebensmittels auf Basis elektromagnetischer Strahlung im sichtbaren Wellenlängenbereich;
Erfassen von Thermografiedaten des Lebensmittels;
Erfassen von Schwingungsdaten, die ein Schwingungsverhalten des Lebensmittels beschreiben;
Ermitteln wenigstens einer Qualitätsgröße für das Lebensmittel auf Basis der Bilddaten, Thermografiedaten und Schwingungsdaten und mittels eines Maschinenlernmodellsystems mit wenigstens einem Maschinenlernmodell.

Das Verfahren kann jeglichen weiteren Schritt umfassen, um sämtliche in dem Zusammenhang mit dem System geschilderten Betriebszustände und/oder Funktionen bereitzustellen. Beispielsweise kann ein Schritt des Positionierens des handhabbaren Teils durch einen Benutzer zum Erfassen zumindest ausgewählter der Sensordaten vorgesehen sein.

Das Verfahren ist typischerweise mit dem beschriebenen System durchführbar bzw. das beschriebene System ist zum Durchführen des beschriebenen Verfahrens eingerichtet.

Die Erfindung betrifft vorzugsweise auch ein Computerprogrammprodukt mit einem Computerprogramm, das Softwaremittel zum Durchführen des beschriebenen Verfahrens oder zum Steuern des beschriebenen Systems aufweist, wenn das Computerprogramm in einem Automatisierungssystem bzw. durch eine Recheneinheit ausgeführt wird.

Ausführungsbeispiele der Erfindung werden im Folgenden anhand der beigefügten schematischen Figuren erläutert. Figurenübergreifend können für gleichartige oder gleichwirkende Merkmale die gleichen Bezugszeichen verwendet werden.
Fig. 1 zeigt den Aufbau eines Systems gemäß einem Ausführungsbeispiel der Erfindung, wobei das System ein Verfahren gemäß einem Ausführungsbeispiel der Erfindung ausführt;
Fig. 2 zeigt eine Prinzipdarstellung eines Maschinenlernmodellsystems, das zum Ermitteln der Qualitätsgröße von dem System aus Figur 1 ausgeführt wird.

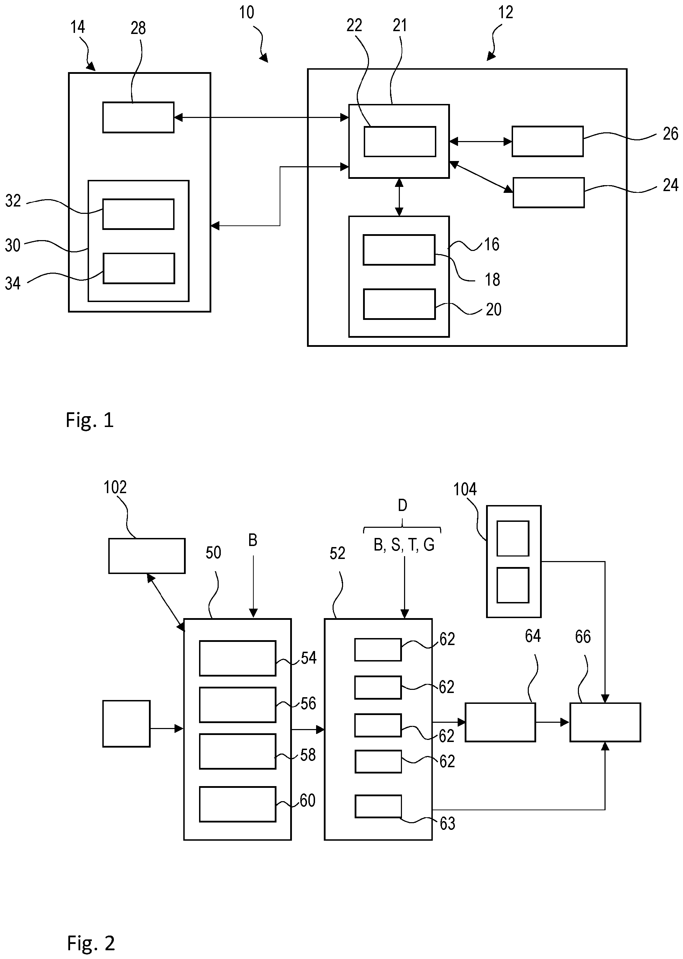

In Fig. 1 ist ein System 10 gemäß einem Ausführungsbeispiel der Erfindung gezeigt. Das System 10 umfasst einen stationären Teil 12, der zum Beispiel auf einem Tisch steht. Ferner umfasst das System 10 einen handhaltbaren und somit bewegbaren Teil 14, den ein Benutzer greifen und aktiv relativ zu dem stationären Teil 12 bewegen kann.

Der stationäre Teil 12 ist insoweit stationär, als dass er zum Überprüfen eines in Fig. 1 nicht gesondert dargestellten Lebensmittels nicht bewegt wird. Es versteht sich aber, dass das gesamte System 10 mobil und insbesondere manuell flexibel transportierbar und aufbaubar ist. Es ist daher nicht z.B. in einer Sortier- oder Verpackungsanlage fest installiert, um dort für einen langfristigen Einsatz zu verbleiben.

In Fig. 1 sind datenübertragende Verbindungen zwischen den einzelnen Einheiten mit Pfeilen eingetragen. Die Datenverbindungen können jeweils kabelgebunden oder kabellos realisiert sein.

Der stationäre Teil 12 umfasst ein Sensorboard 16, das in einen nicht gesondert dargestellten Auflagebereich zum Auflegen des zu prüfenden Lebensmittels integriert ist. Das Sensorboard 16 umfasst einen Gewichtssensor 18 und einen Umgebungssensor 20, der ein Thermo-Hygrometer ist.

Weiter umfasst der stationäre Teil 12 eine Ermittlungseinrichtung 21, die wenigstens einen Prozessor 22 zum Ausführen des Maschinenlernmodellsystems umfasst. Die Ermittlungseinrichtung 21 bzw. deren Prozessor 22 kann den allgemeinen Betrieb des Systems 10 steuern und sämtliche hierin geschilderten Arbeitsabläufe und Systemfunktionen steuern und/oder ausführen.

Optional weist das System 10 auch eine Eingabe-/Ausgabeeinrichtung 24 auf, die eine Anzeigeeinrichtung und wenigstens eine Eingabeeinrichtung zum Erhalten von Nutzerumgaben umfasst. In einer Variante ist die Eingabe-/Ausgabeeinrichtung 24 ein berührungsempflindlicher Bildschirm. Im gezeigten Fall ist die Eingabe-/Ausgabeeinrichtung 24 Bestandteil des stationären Teils 12, kann aber ebenso von dem bewegbaren Teil 14 umfasst sein.

Schließlich umfasst der stationäre Teil 12 einen Thermografiesensor 26 in Form einer Wärmebildkamera, deren Erfassungsbereich auf den Auflagebereich gerichtet ist, um ein dort positioniertes Lebensmittel zu erfassen. Da es sich um einen aktiven Thermografiesensor 26 handelt, umfasst dieser auch eine nicht gesondert dargestellte Wärmequelle in Form einer Blitzlampe.

Der bewegbare Teil 14 umfasst einen Kamerasensor 28 zum Erfassen von Bilddaten des Lebensmittels auf Basis von sichtbarem Licht.

Ferner umfasst der bewegbare Teil 14 eine Einrichtung 30 zum Erfassen des Schwingungsverhaltens des Lebensmittels. Diese Einrichtung 30 umfasst ein Mikrofon 32 und einen Aktor 34 (insbesondere umfassend einen Stößel) zum Erzeugen einer Schwingungsanregung.

Zum Überprüfen eines Lebensmittels, das im gezeigten Beispiel von Obst oder Gemüse ist, positioniert der Benutzer das Obst oder Gemüse in dem Auflagebereich, woraufhin dessen Gewicht und Wärmebild erfasst wird. Der Umgebungssensor 20 erfasst zusätzlich die Temperatur und Luftfeuchtigkeit. Sämtliche erfassten Daten und Informationen werden an die Ermittlungseinrichtung 21 gesendet.

Der Benutzer positioniert den beweglichen Teil 14 derart, dass mit der Kamera 28 ein Bild des Lebensmittels erfassbar ist und löst die Bilderfassung aus. Weiter hält er den bewegbaren Teil 14 an das Lebensmittel, um mit den Aktor 34 eine Schwingungsanregung an das Lebensmittel zu übertragen und die Schwingungen des Lebensmittels in Form akustischer Schwingungssignale mit dem Mikrofon 32 zu erfassen. Die auf die Weise erhaltenen Schwingungsdaten und Bilddaten werden ebenfalls an die Ermittlungseinrichtung 21 übersandt.

Die Ermittlungseinrichtung 21 ermittelt auf Basis der insgesamt erhaltenen Daten als Qualitätsgrößen einen Reifegrad des Lebensmittels und eine voraussichtliche Haltbarkeit. Dies wird anhand von Fig. 2 näher erläutert.

Fig. 2 zeigt eine Prinzipdarstellung eines Maschinenlernmodellsystems 100 oder, mit anderen Worten, ein Funktionsschema eines Computeralgorithmus oder Computerprogramms zum Implementieren eines Maschinenlernmodellsystems 100. Das Maschinenlernmodellsystem 100 bzw. der Computeralgorithmus wird von dem Prozessor 22 der Ermittlungseinrichtung 21 ausgeführt. Das Computerprogramm umfasst ein Maschinenlernmodellsystem 100 mit einer Mehrzahl von Maschinenlernmodellen in Form künstlicher neuronaler Netzwerke. Folglich kann auch von einem Cluster an Maschinenlernmodellen gesprochen. Diese sind in der nachstehend geschilderten Weise zum Teil miteinander verknüpft, indem z.B. deren Ermittlungsergebnisse fusioniert werden.

Gezeigt sind ein erstes Modul 50 und ein zweites Modul 52 des Maschinenlernmodellsystem 100, die jeweils eine Mehrzahl von einzelnen Maschinenlernmodellen umfassen. Das erste Modul 50 erhält bevorzugt als Eingangsdaten lediglich die Bilddaten B des Kamerasensors 28, kann optional jedoch auch jegliche der vorstehend geschilderten anderweitigen Sensordaten erhalten.

Auf Basis der Bilddaten B erfolgt mittels eines ersten Maschinenlernmodells 54 eine Klassifizierung des Lebensmittels, beispielsweise durch Ermitteln der übergeordneten Klasse (z. B. Obst, Gemüse, Fisch, Fleisch) und/oder einer genauen Lebensmittelsorte (z.B. der Obst- oder Gemüsesorte). Hierdurch wird das Anwendungsspektrum erhöht, da wie noch geschildert, auf Basis dieses Klassifikationsergebnisses wenigstens ein klassen- und/oder sortenspezifisches Maschinenlernmodell bedarfsgerecht ausgewählt werden kann.

Gemäß einem allgemeinen und nicht auf das Ausführungsbeispiel beschränkten Aspekt, kann das System 10 eine Mehrzahl von klassen- und/oder sortenspezifischen Maschinenlernmodellen aufweisen. Aus diesen kann anhand des Klassifikationsergebnisses eine Auswahl getroffen werden.

Mittels eines zweiten Maschinenlernmodells 56 wird eine Objekterkennung durchgeführt, wobei die Objekte vorbestimmte Fehler und Abweichungen auf der Lebensmitteloberfläche sind, beispielsweise Druckstellen, Kratzer oder Schimmel. Mittels eines dritten Maschinenlernmodells 58 werden morphologische Eigenschaften des Lebensmittels ermittelt, wie dessen Länge oder Durchmesser. Ein viertes Maschinenlernmodells 60 rekonstruiert verdeckte Flächen des Lebensmittels, beispielsweise falls dessen Oberfläche teilweise durch Aufkleber oder anderweitige Fremdobjekte verdeckt ist.

Sämtliche der erhaltenen Ermittlungsergebnisse können mittels der Eingabe-Ausgabeeinrichtung 24 ausgegeben werden. Bevorzugt erfolgt dies zumindest für das Klassifizierungsergebnis und/oder die rekonstruierte Oberfläche. Auch die ermittelten Abweichungen der Lebensmitteloberfläche können dem Benutzer unmittelbar angezeigt werden.

An das zweite Modul 52 werden jedoch bevorzugt nur ausgewählte Ermittlungsergebnisse übergeben (siehe Pfeil in Figur 2), beispielsweise das Klassifizierungsergebnis oder morphologische Eigenschaften wie das Volumen. Diese können dort als Hilfsvariablen oder "auxiliary variables" der dortigen neuronalen Netze verwendet werden. Diese Hilfsvariable wird typischerweise nach der Komprimierung der Information der akustischen Signale verwendet, in der Regel entweder nach einer "Global Average Pooling" oder "Flattening Layer". Die Verknüpfung der Hilfsvariable mit den extrahierten Informationen der akustischen Signale erfolgt bei uns durch eine Konkatinierung, kann aber auch durch andere Operationen wie "Add", "Merge" oder "Dot" erfolgen. Jedoch werden vorzugsweise Informationen betreffend ermittelte Abweichungen der Lebensmitteloberfläche nicht an das zweite Modul 52 übermittelt. Hintergrund ist, dass diese Abweichungen nicht immer zwingend einem Qualitätsmangel entsprechen, weshalb der Benutzer die Beurteilung, ob es sich um einen Qualitätsmangel handelt oder nicht, selbst vornehmen sollte.

Das zweite Modul 52 erhält neben den vorstehenden Hilfsvariablen sämtliche Sensordaten D als Eingangsgröße, also die Bilddaten B, die Thermografiedaten T, die Gewichtsdaten G und die Schwingungsdaten S.

Für jeden Typ dieser Sensordaten D existiert ein einzelnes neuronales Netz 62, das die entsprechenden Sensordaten D dieses Typs als Eingangsgröße erhält. Das jeweilige neuronale Netz 62 führt einen Klassifizierungsvorgang durch, um als eine erste Qualitätsgröße einen Reifegrad (zum Beispiel unreif, reif, überreif) des Lebensmittels zu bestimmen. Ebenso führt jedes sensordatenspezifische neuronale Netz 62 einen Regressionsvorgang durch, um als eine weitere Qualitätsgröße die Haltbarkeit (und bevorzugt als eine noch weitere Qualitätsgröße auch den Zuckergehalt) des Lebensmittels vorherzusagen. Um diese Qualitätsprüfung vorzunehmen, wurden die jeweiligen neuronalen Netze 62 mittels Trainingsdaten trainiert, die einen verifizierten Zusammenhang zwischen den jeweiligen Sensordaten D und diesen Qualitätsgrößen abbilden.

Die jeweils erhaltenen Regressions- und Klassifikationsergebnisse werden in einem Funktionsblock 64 fusioniert, um ein Gesamtergebnis der gesuchten Qualitätsgrößen zu erhalten. Für diese Daten- bzw. Ergebnisfusion können gängige Ansätze des Standes der Technik gewählt werden.

Die neuronalen Netze 62 können Bestandteil eines allgemeine Obst- oder Gemüseclusters sein, das unabhängig von der genauen Obst- oder Gemüsesorten stets zur Qualitätsgrößenermittlung verwendet wird. Anhand des Klassifikationsergebnisses des ersten Moduls 50 kann dieses Obst- oder Gemüsecluster zum Beispiel anstelle eines Fleischclusters ausgewählt werden.

Alternativ zu dem beschriebenen Verwenden sensordatenspezifischer neuronaler Netz 62 mit anschließender Ergebnisfusion kann wenigstens ein neuronales Netz 62 mehrere verschieden Arten von Sensordaten D als Eingangsgrößen erhalten, können also zumindest Teile der Sensordaten D vorab oder innerhalb eines neuronalen Netzes 62 fusioniert werden. Mit der hier gewählten erst nachträglichen Ergebnisfusion wird aber eine verbesserte Erklärbarkeit des Maschinenlernmodellsystems 100 nach erfolgtem Trainieren erhalten. Die verbesserte Erklärbarkeit ermöglicht u.a. ein zielgerichtetes Training, insbesondere falls eine gewünschte Ermittlungsgenauigkeit zunächst nicht erzielt wird.

Als eine bevorzugte Variante ist noch wenigstens ein weiteres neuronales Netz 63 vorgesehen, das eine lebensmittelartspezifische Qualitätsgrößenermittlung durchführt. Dieses neuronale Netz 63 kann ebenfalls anhand des Klassifikationsergebnisses des ersten Moduls 50 ausgewählt werden. Genauer gesagt kann dieses neuronale Netz 63 eine z.B. gemüsesorten- oder obstsortenspezifische Qualitätsgröße ermitteln. Im Fall einer Wassermelone kann die sortenspezifische Qualitätsgröße z.B. eine Schalendicke sein. Dieses neuronale Netz 63 kann Bestandteil eines arten- bzw. sortenspezifischen Clusters sein.

Die insgesamt ermittelten Qualitätsgrößen werden im Block 66 optional hinsichtlich deren vertrauenswürdig geprüft. Hierfür kann gemäß dem Block 104 eine erklärbare künstliche Intelligenz Funktion 106 und/oder eine AlOps (engl.: artificial intelligence for IT operations; deutsch: künstliche Intelligenz für IT-Operationen) Funktion 108 verwendet werden. Bevorzugt weist das Maschinenlernmodellsystem 100 auch eine aktive Lernfunktion 102 auf. Diese ermöglicht das Anfordern von Benutzereingaben, um z.B. im laufenden Betrieb verifizierte Datensätze für nur unsicher oder auch gar nicht klassifizierbare Lebensmittel zu erhalten. Die angeforderten Benutzereingaben können insbesondere eine der folgenden betreffen: Ob es ich um eine Frucht mit oder ohne Schale/Rinde handelt; Ob aktuell eine Hauptreifezeit der Frucht vorliegt; Eine Angabe der Herkunft der Frucht; Eine Bezeichnung der Frucht.

Im Rahmen eines Trainings des Maschinenlernmodellsystems 100 und insbesondere der neuronalen Netze 62, 63 des zweiten Moduls 52 kann ein sogenanntes gemeinschaftliches Lernen (engl.: ensemble learning) zum Einsatz kommen. Allgemein kann das Maschinenlernmodellsystem 100 als ein System aus einer Mehrzahl von individuell trainierten Deep-Learning-Architekturen verstanden werden.

## Patentansprüche

1. System (10) zur manuellen Qualitätsprüfung von Obst und Gemüse und anderen Lebensmitteln, mit:
- einem Kamerasensor (28) zum Erfassen von Bilddaten (B) des Lebensmittels auf Basis elektromagnetischer Strahlung im sichtbaren Wellenlängenbereich;
- einem Thermografiesensor (26) zum Erfassen von Thermografiedaten (T) des Lebensmittels;
- einer Einrichtung (30) zum Erfassen von Schwingungsdaten (S), die ein Schwingungsverhalten des Lebensmittels beschreiben;
- wenigstens einer Ermittlungseinrichtung (21), die dazu eingerichtet ist, auf Basis der Bilddaten (B), Thermografiedaten (T) und Schwingungsdaten (S) sowie mittels Maschinenlernmodellsystems (100) mit wenigstens einem Maschinenlernmodell (54-60, 62, 63) eine Qualitätsgröße für das Lebensmittel zu ermitteln;
wobei das System (10) einen handhaltbaren Teil (14) und einen stationären Teil (12) umfasst, wobei der handhaltbare Teil (14) aktiv relativ zu dem Lebensmittel und zu dem stationären Teil (12) beweglich ist,
und wobei der handhaltbare Teil (14) zumindest einen von dem Kamerasensor (28), dem Thermografiesensor (26) und der Einrichtung (30) zum Erfassen der Schwingungsdaten (S) aufweist.

2. System (10) nach Anspruch 1,
ferner **gekennzeichnet durch** einen Umgebungssensor (20) zum Erfassen wenigstens einer Umgebungsgröße, vorzugsweise der Temperatur oder der Luftfeuchtigkeit, und/oder durch einen Gewichtssensor (18) zum Erfassen des Gewichts des Lebensmittels,
wobei die Qualitätsgröße auch auf Basis Erfassungsdaten von dem Umgebungssensor (20) und/oder dem Gewichtssensor (18) ermittelt wird.

3. System (10) nach Anspruch 2,
**dadurch gekennzeichnet, dass** der Umgebungssensor (20) und/oder der Gewichtssensor (18) von dem stationären Teil (12) umfasst sind.

4. System (10) nach einem der vorangehenden Ansprüche,
**dadurch gekennzeichnet, dass** die Einrichtung (30) zum Erfassen der Schwingungsdaten (S) einen Aktor (34) zum Ausüben eines Stoßes auf das Lebensmittel und einen Mikrofonsensor (32) zum Erfassen akustischer Signale des Lebensmittels infolge des Stoßes umfasst.

5. System (10) nach einem der vorangehenden Ansprüche,
**dadurch gekennzeichnet, dass** das Maschinenlernmodellsystem (100) wenigstens ein erstes Modul (50) für eine Grobprüfung des Lebensmittels und wenigstens ein zweites Modul (52) für das Ermitteln der Qualitätsgröße aufweist.

6. System (10) nach einem der vorangehenden Ansprüche,
**dadurch gekennzeichnet, dass** das Maschinenlernmodellsystem (100) eine Mehrzahl von Maschinenlernmodellen (54-60, 62, 63) umfasst, von denen wenigstens eines dazu eingerichtet ist, auf Basis nur einer ausgewählten Art der Sensordaten (B, T, S) ein Ergebnis zu ermitteln, das mit einem ermittelten Ergebnis wenigstens eines anderen Maschinenlernmodells (54-60, 62, 63) des Maschinenlernmodellsystems (100) zum Bestimmen der Qualitätsgröße fusionierbar ist.

7. System (10) nach einem der vorangehenden Ansprüche,
**dadurch gekennzeichnet, dass** die Qualitätsgröße einen Reifegrad des Lebensmittels angibt und/oder die Qualitätsgröße eine voraussichtliche Haltbarkeit des Lebensmittels angibt.

8. System (10) nach einem der vorangehenden Ansprüche,
**dadurch gekennzeichnet, dass** die Ermittlungseinrichtung (21) dazu eingerichtet ist, die Qualitätsgröße auf Basis eines Klassifikationsvorgangs und/oder eines Regressionsvorgangs zu ermitteln, wobei der Klassifikationsvorgang und/oder der Regressionsvorgang jeweils unter Verwendung des Maschinenlernmodellsystems (100) durchführbar sind.

9. System (10) nach einem der vorangehenden Ansprüche,
**dadurch gekennzeichnet, dass** die Ermittlungseinrichtung (21) dazu eingerichtet ist, auf Basis der Bilddaten (D) wenigstens einen der folgenden zu ermitteln:
einen Ort des Lebensmittels, an dem zum Erfassen des Schwingungsverhaltens eine Schwingungsanregung erfolgt;
einen Ort des Lebensmittels, an dem dieses eine vorbestimmte unnatürliche Oberflächenabweichung aufweist, beispielsweise einen Aufkleber oder eine Markierung;
einen Ort des Lebensmittels, an dem dieses einen vorbestimmten natürlichen Oberflächenfehler aufweist, beispielsweise einen Kratzer, Schimmel oder eine Druckstelle.

10. Verfahren zur Qualitätsprüfung von Obst und Gemüse und anderen Lebensmitteln, mit:
- Erfassen von Bilddaten (B) des Lebensmittels auf Basis elektromagnetischer Strahlung im sichtbaren Wellenlängenbereich;
- Erfassen von Thermografiedaten (T) des Lebensmittels;
- Erfassen von Schwingungsdaten (S), die ein Schwingungsverhalten des Lebensmittels beschreiben;
- Ermitteln wenigstens einer Qualitätsgröße für das Lebensmittel auf Basis der Bilddaten (B), Thermografiedaten (T) und Schwingungsdaten (S) und mittels eines Maschinenlernmodellsystems (100) mit wenigstens einem Maschinenlernmodell (54-60, 62, 63).
